# EUROPEAN PATENT SPECIFICATION

(11) **EP 0 684 808 B1**
(45) Date of publication and mention of the grant of the patent: **10.12.1997**
(21) Application number: 94910158.8
(22) Date of filing: 15.02.1994
(51) Int. Cl.: A61K 7/00, A61K 7/42, C09B 67/00

(54) **LIGHT STABLE COLOR COMPOSITIONS**
LICHTSTABILE FARBZUSAMMENSETZUNGEN
COMPOSITIONS COLORANTES STABLES A LA LUMIERE

(30) Priority: 16.02.1993 US 18089
(43) Date of publication of application: 06.12.1995
(73) Proprietor: WHITTAKER, CLARK & DANIELS, INC., South Plainfield, NJ 07080-1000 (US)
(72) Inventor: LAMBRIDIS, George, Wayne, NJ 07470 (US); GOODWIN, Nathanial, Newark, NJ 07108 (US)
(74) Representative: Schwarz, Albin, Dr. Kopecky & Schwarz Patentanwälte
(86) International application number: PCT/US94/01929
(87) International publication number: WO 94/18932

(56) References cited:
- EP-A- 0 220 617
- EP-A- 0 328 906
- WO-A-86/04598
- WO-A-90/06103
- FR-A- 2 134 621
- US-A- 4 648 908
- PATENT ABSTRACTS OF JAPAN vol. 13, no. 84 (C-572)27 February 1989 & JP,A,63 270 618 (KANEBO LTD) 8 November 1988
- DATABASE WPI Section Ch, Week 7715, Derwent Publications Ltd., London, GB; Class D11, AN 77-26389Y & JP,A,52 009 442 (SHISEIDO KK) 16 March 1977

## Description

The present invention relates to color compositions which have improved light stability and sun protection factor (SPF) properties. More specifically, the compositions of the present invention have an outer protective layer which absorbs ultraviolet light and thereby prevents the fading or discoloration of the colorant- containing particles. The outer layer further acts to impart an improved sunburn protection factor to the novel compositions of the invention.

### Background of the Invention

Color compositions are widely used in the cosmetic industry principally for aesthetic purposes. Document FR-A-2 134 621 discloses particles of TiO₂ pigments coated with propylene glycol and metal oxides. Accordingly, it is important that color compositions remain stable, i.e., not fade or discolor. One principal cause for discoloration of color compositions used in the cosmetic field is the tendency of colorants to absorb light, principally ultraviolet light, which results in the degradation of the colorant molecules.

In the past, efforts to improve the light stability of color compositions have involved generally the use of ultraviolet light absorbing compositions, such as benzophenones, and/or antioxidants, such as butylated- hydroxytoluene (BHT) or butylated hydroxyanisol (BHA).

An object of the present invention is to provide compositions comprising colorant-containing particles with improved light stability properties so that these compositions as well as cosmetic products which include them are less susceptible to fading or discoloration.

Another object of the present invention is to provide color compositions having an improved sunburn protection factor (SPF).

Yet a further object of the invention is to provide a process for making color compositions which have improved light stability and SPF properties.

These and further objects and advantages of the present invention will, upon reading the following specification, become apparent to those skilled in the field of color compositions.

### Summary of the Invention

It has been found that the objects and advantages described above are realized by compositions comprising colorant-containing particles which are coated with a composition comprising a suitable carrier component, such as titanium dioxide (TiO₂), and a propylene glycol component. This coating renders colorant-containing substrate particles resistant to degradation by ultraviolet light and is therefore particularly useful for light sensitive colorants. Although carriers other than TiO₂ may be used as a coating component, the invention will be primarily described with reference to TiO₂. Accordingly, one aspect of the present invention is the novel color compositions comprising colorant-containing particles coated with titanium dioxide and propylene glycol. Another aspect of the invention is the novel process for preparing the coated color containing particles. A further aspect of the invention is the novel use of carriers such as TiO₂, in combination with propylene glycol to impart the property of improved light stability to the colorant-containing particles. And yet a further aspect of the invention is the novel use of TiO₂ and propylene glycol to impart an improved SPF property to compositions comprising colorant-containing particles. A still further aspect of the invention is the use of novel substrate molecules, including but not limited to starch and mica, in coated colorant-containing particles. Yet another aspect of the invention is the use of propylene glycol to improve the SPF of micronized TiO₂.

### Brief Description of the Drawings

Figure 1 shows SPF results for micronized TiO₂.

Figure 2 shows SPF results for micronized TiO₂ and 5% by weight of propylene glycol.

Figure 3 shows color spectrophotometric measurements for FD&C blue #1 dye laked onto starch (B1) or aluminum hydrate (B2).

Figure 4 shows color spectrophotometric measurements for FD&C green #5 laked onto starch (G1) or aluminum hydrate (G2).

Figure 5 shows color spectrophotometric measurements for D&C red #28 laked onto starch (R1) or aluminum hydrate (R2).

### Detailed Description of the Invention

The present invention has been found to significantly improve both light stability and SPF properties of compositions comprising colorant-containing particles which are unstable in ultraviolet light. While such color compositions are used in a wide number of fields, e.g., paints and cosmetics, the following discussion will primarily be addressed to compositions for use in the cosmetic field; notwithstanding the fact that application of this invention embraces any and all fields where the stability of color compositions is desirable.

In accordance with the present invention, colored compositions are prepared by coating colorant-containing particles with a composition comprising propylene glycol and carrier particles.

As used herein, the term colorant-containing particles refers to any particle which imparts color useful in cosmetic, paint or similar compositions and particularly those which are light sensitive, i.e., susceptible to fading or discoloration. While colorant-containing particles include particles of inorganic pigments, e.g., iron oxides, the preferred colorant-containing particles comprise organic dyes laked onto a suitable substrate such as TiO₂, and other such FD&C and D&C approved substrates. Specifically the dyes include, but are not limited to, the well-known FD&C and D&C dyes, for example, FD&C Blue No. 1, FD&C Yellow No. 5, FD&C Green No. 5, Brown 13693, carmoisine edicol, FD&C Red No. 40, D&C Red 21, D&C Red 27 and D&C Red 28.

Hereinafter the colorant, unless indicated otherwise, will refer to FD&C and D&C dyes laked on to a suitable substrate in accordance with conventional dilution or slurry methods.

FD&C, D&C and External D&C dyes may be used to make insoluble lake pigments. The formation of a lake pigment requires three basic conditions; namely, (i) a water soluble dye; (ii) a suitable substrate; and (iii) a suitable precipitating agent. The laking process involves the following steps: (1) addition of a substrate; (2) addition of a suitable dye; (3) absorption of the dye by the substrate; (4) addition of a metal salt; and (5) formation and precipitation of a lake pigment. Once the laking process is completed, the water soluble dyes are converted to water insoluble pigments. The water soluble dye will impart the basic color hue. The substrate will influence the transparency, opacity, brightness, etc.

While TiO₂ is a preferred substrate onto which the dye or suitable mixtures thereof is absorbed, other substrates include all FD&C and D&C approved substrates e.g., alumina, zinc oxide, aluminum benzoate, calcium carbonate, and those listed in the Code of Federal Regulations, Food and Drugs, paragraph 82.1051, subpart C - Drugs and Cosmetics, or combinations of such substrates, as well as, according to the present invention, substrates which have not yet been approved but which exhibit suitable physical properties, such as mica or starch.

It has been found that when FD&C or D&C dyes were laked on to a starch substrate, pigment laked with more transparency, better light reflectance, higher color value, better feel and good light stability. Suitable starches include, but are not limited to, food grade starches such as starch derived from cornmeal and/or tapioca. Suitable dyes include, but are not limited to, FD&C Blue No. 1, D&C Red No. 28, FD&C Green No. 5, and FD&C Yellow No. 5. Although not yet an FDA approved substrate, the favorable physical properties of starch indicate that it may be desirable to petition the FDA to approve its use in cosmetics and drugs. Alternatively, mica may be used as substrate.

Colorant-containing particles can be formed by absorbing an organic dye onto substrate particles which are preferably in the form of micronized particles (average particle size of .05 to .4 µm). The resulting particles are then advantageously precipitated, dried and crushed to form a fine powder. The particle size depends on the type of pulverizing equipment used; however a particle size of about .2 to .3 µm is preferred and is obtained by using a Model No. 2D4 Pulverizer. TiO₂ is a preferred substrate for the colorant because of its superior sunscreen properties. However, since TiO₂ absorbs ultraviolet radiation, its use contributes to the degradation of the absorbed colorant (dye) which results in its fading and/ or discoloration.

The light stability of colorants, particularly those absorbed on a TiO₂ substrate, is remarkably improved by coating the colorant particle with a coat comprising a carrier component, such as TiO₂, and propylene glycol. While the carrier TiO₂ is a preferred component of the coating composition, other suitable coating components include carriers such as zinc oxide and aluminum hydroxide. The carrier component is preferably micronized, i.e., having a particle size in the range of about .1 to about .3 µm. While these are the generally preferred parameters for the particle size of the carrier component, non-micronized particles may be used; however, the size of particles in the coating must, of course, be substantially smaller than the size of the colorant-containing particle onto which it is to be coated.

In addition to the carrier component, the coating composition further comprises propylene glycol. As will be apparent from the examples set forth below, the propylene glycol is preferably sprayed onto a homogeneous mixture of the colorant-containing particles and the micronized carrier. Nevertheless, however the propylene glycol is added to the mixture, it is desirable that the homogeneous mixture is uniformly contacted with propylene glycol and that the particles do not agglomerate when contacted with the propylene glycol. By adding to the colorant-containing particles a coating comprising a micronized carrier or mixtures thereof and the propylene glycol component, the coating (i) acts as a protective barrier against radiation (which causes fading and discoloration) reaching the colorant-containing particles and (ii) increases the sun protection factor (SPF) of compositions containing the particles.

The coating composition used in the present invention preferably contains the propylene glycol and the carrier component in a ratio of from about 1:4 to about 1:5 by weight. However, other suitable ratios of propylene glycol and carrier may be used. The ratio of coating composition to colorant-containing particle used to coat colorant-containing particles depends on the shade of the colorant which is desired, i.e., the more coating that is used the paler the shade of the colorant. The size of the resulting coated colorant-containing particles depends on both the initial particle size and the coating thickness. Nevertheless, generally final particle sizes of about 1 µm or less are suitable and micronized particle sizes are preferred especially for cosmetic uses, i.e., particularly for use as a sunscreen factor. The present invention also provides for a method of improving the sun protection factor of a composition comprising sunscreen grade TiO₂ (micronized TiO₂ with a particle size of 1 µm or less) comprising coating the TiO₂ particles with propylene glycol as well as providing for compositions produced by this method.

In specific, non-limiting embodiments of the present invention, the photostability of FD&C and D&C dyes laked onto a substrate particle can be significantly improved by contacting the laked substrate particle with a coating containing propylene glycol as well as particles of the compound used as substrate, in uncolored form and at a suitable size. For example, and not by way of limitation, the photostability of dye laked on TiO₂ substrate particles may be improved by coating the dye-laked TiO₂ particles with uncolored TiO₂ particles and propylene glycol; the photostability of dye laked on zinc oxide substrate particles may be improved by coating the dye-laked zinc oxide particles with uncolored zinc oxide particles and propylene glycol; and the photostability of dye laked on starch or mica, may be improved by coating the dye-laked starch or mica particle with uncolored starch or mica particles and propylene glycol.

The following examples are provided in order to disclose the invention in the fullest possible extent and in the best mode known. Nevertheless they are to be construed as purely illustrative and not limiting of the present invention.

### Example 1

### Procedure

In order to make 908 g of uncoated light stable micronized TiO₂ colored with FD&C Blue No. 1, the following procedure was followed:

The color load chosen was 1% by weight.

The 9.08 g of FD&C Blue No. 1 were dissolved in 3,500 ml of water. The solution was mixed well, until all the dye was completely dissolved. In a separate container, 10,000 ml of water was added and vigorously mixed, so as to slowly disperse 726 g of micronized TiO₂ having an average particle size of .1 µm. The dispersion was mixed for 15 minutes until dispersed completely using Yamato LR-41A Labo-Stirrer. Thereafter the dye solution (FD&C Blue No. 1) was added to the TiO₂ dispersion while mixing, and then continued to be mixed for 30 minutes to allow all the color to be absorbed by the TiO₂ particles. The volume was thereafter increased to 16,000 mls by the addition of tap water. The colorant-particle solution was then precipitated out by using an AlCl₃ solution, 32° Bé, diluted 1:1 with water.

After adding the AlCl₃ solution, the pH was checked and if necessary adjusted to be 3.0 ± 0.3 with AlCl₃. Also, the bleed was checked, i.e., to determine the absence of unprecipitated dye, and once it was determined to be negative, the precipitant was filtered using a Buchner filtration apparatus with a whatman qualitative #5 filter. The filtered product was dried in a Lindberg/Bleum laboratory oven Model No. SW-17TA-1 at a temperature between 70°-80°C. when fully dried, i.e., all moisture absent, the product was crushed and pulverized in a pulverizer Model No. 204 to a fine particle size having an average of about .2 to .3 µm.

The uncoated color particles were then placed in a Waring CB6 blender (Model No. 34 BL 22) and 182 gms. of micronized TiO₂ were added. The mixture was blended well until homogeneous and uniform. While blending, 136.2 g of propylene glycol were uniformly sprayed (using a True-Temper pressure sprayer Model No. HS-200) onto the batch carefully so as to avoid any agglomeration of the particles and the batch was well blended until homogenous and uniform.

### Example 2

An alternative method of preparing uncoated particles of the colored TiO₂ lake is the slurry method. According to this method, 310 g of micronized TiO₂ (having an average particle size of about .1 µm) are dispersed in 500 g of water using a homogenizer. This mixture is homogenized until a low to medium viscosity suspension is obtained.

2.5 g. of a suitable FD&C (or D&C) dye is dissolved in 50 g of water, added to the blend, and mixed using a paddle mixer for 30 minutes until all the dye is absorbed by the TiO₂.

The dye is then precipitated with an AlCl₃ solution. Once the bleed is determined to be negative, the slurry is transferred and dried at 70°-80°C. When all the water has evaporated, the product is crushed and pulverized to a small particle size (having an average size of about .2 to .3 µm). Thereafter a micronized TiO₂ /propylene glycol coat is added, first by adding 55.14 g micronized TiO₂ (average particle size .1 µm) and, once homogeneous and uniform, 18.38 g propylene glycol is uniformly sprayed onto the batch and the batch is mixed well until uniform. The light stability of the product made with the slurry method is similar to that obtained in Example 1.

### Example 3

Samples of both the uncoated colorant particles and coated colorant particles prepared in accordance with Example 1 were placed in 2.96·10⁻⁵m³ clear plastic vials and exposed for 24 hours to the following sources of radiation:
- shortwave ultraviolet
- longwave ultraviolet
- white light 3600° Kelvin (approximate)
- north sky light 7000° K (approximate)

After the 24 hour exposure, samples of the uncoated colorant particles showed, in all cases, complete fading and developed an off-white to gray-white color. The samples of the coated colorant particles, i.e., those which were coated with the micronized TiO₂ and propylene glycol showed none to slight color loss, maintaining the characteristics of their original color.

Additional tests were performed using other dyes such as FD&C Yellow No. 5, Brown 13693, and carmoisine edicol, and all showed the same results as the FD&C Blue No. 1.

### Example 4

The SPF effectiveness of the micronized TiO₂/propylene glycol coated titanium dioxide particles was established by comparing the following two samples:
- micronized TiO₂ (Sample 1)
- micronized TiO₂ and 5% by weight of propylene glycol (Sample 2)

The latter sample was prepared by spraying 95 g. of micronized TiO₂ (average particle size of .1 µm) with 5 g. of propylene glycol and mixing the sprayed TiO₂ particles well to insure a homogeneous mixture.

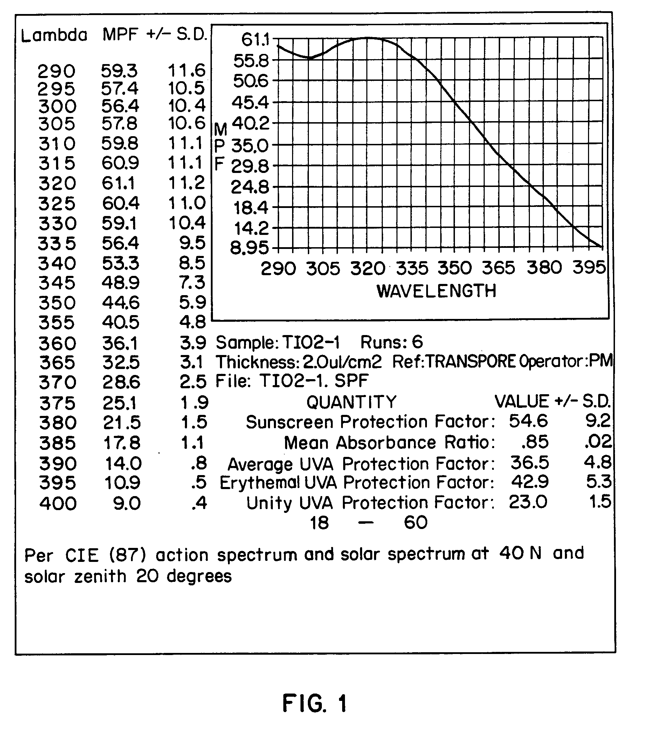

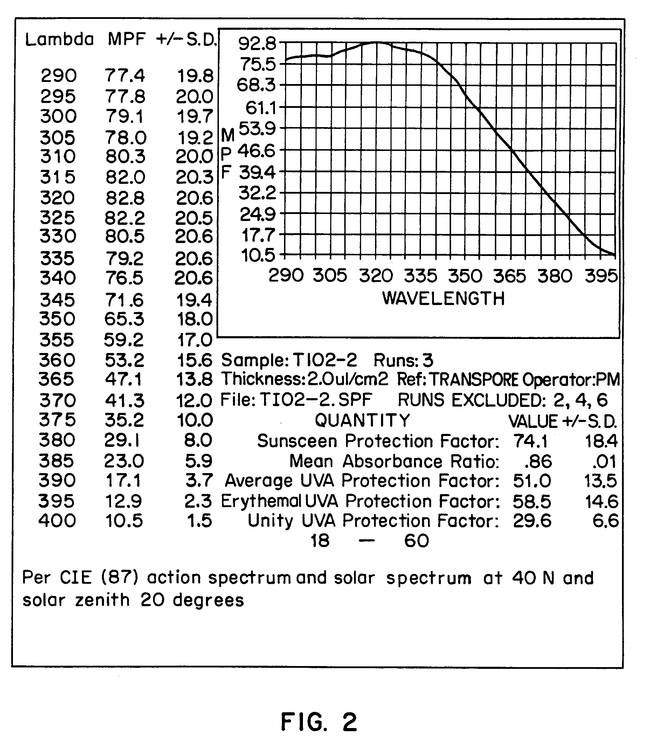

A 20% dispersion of each of the above samples in isopropyl myristate was prepared. Each sample was thereafter measured to determine its SPF (sun protection factor) using a SPF-290 spectrophotometer (Optometrics USA). The results are shown in Figures 1 and 2. Figure 1 shows SPF results for sample 1. Figure 2 shows SPF results for sample 2.

By comparing the two graphs, it was concluded that the addition of 5% propylene glycol to the TiO₂ particles significantly increased the SPF value of the TiO₂ when used as a sunscreen agent. It should be noted that during the measurement, six runs were taken on each sample. Because of the fact that for the TiO₂/propylene glycol (Sample 2) the average was off the instrument's scale, runs 2, 4, and 6 were excluded in order to plot a curve within the instrument's capability (maximum 99.99).

### Example 5

The following experiments were performed in order to evaluate the usefulness of starch as a substrate for laked pigment.

Blue, green, or red dye was laked onto either starch or aluminum hydrate substrates, and the quality of color associated with the laked pigments were compared spectrophotometrically.

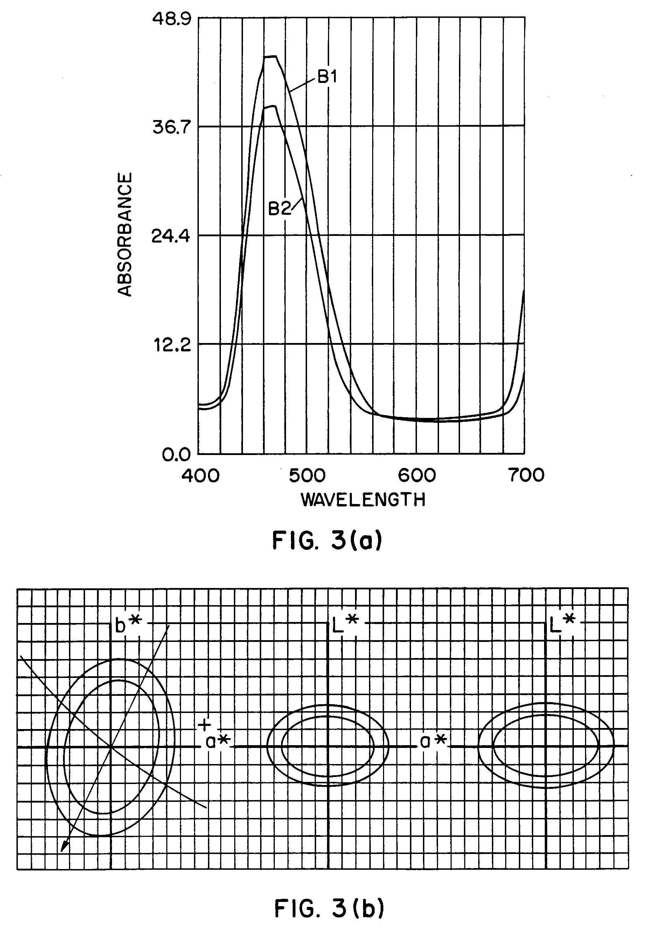

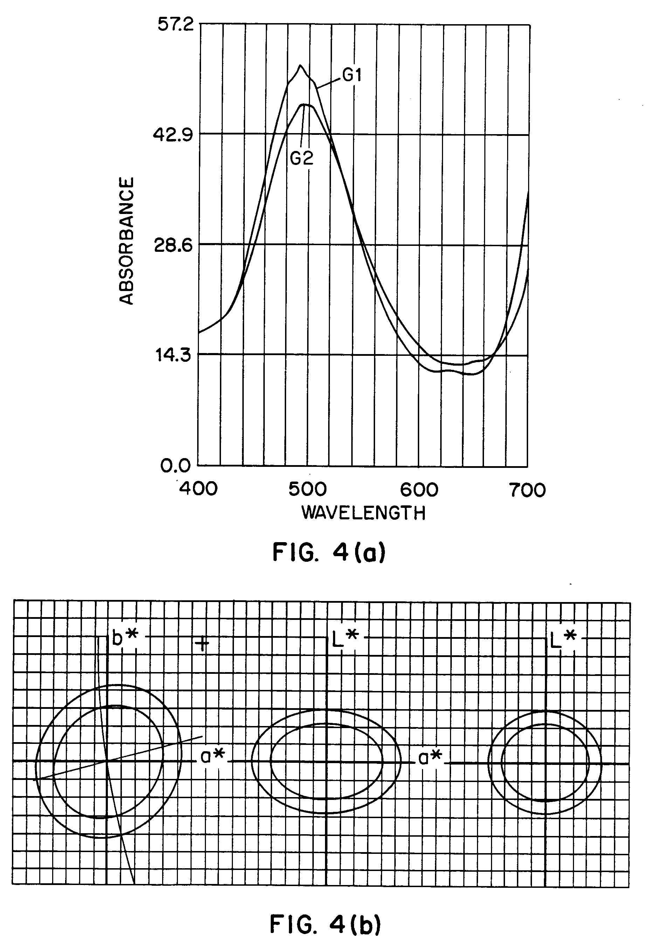

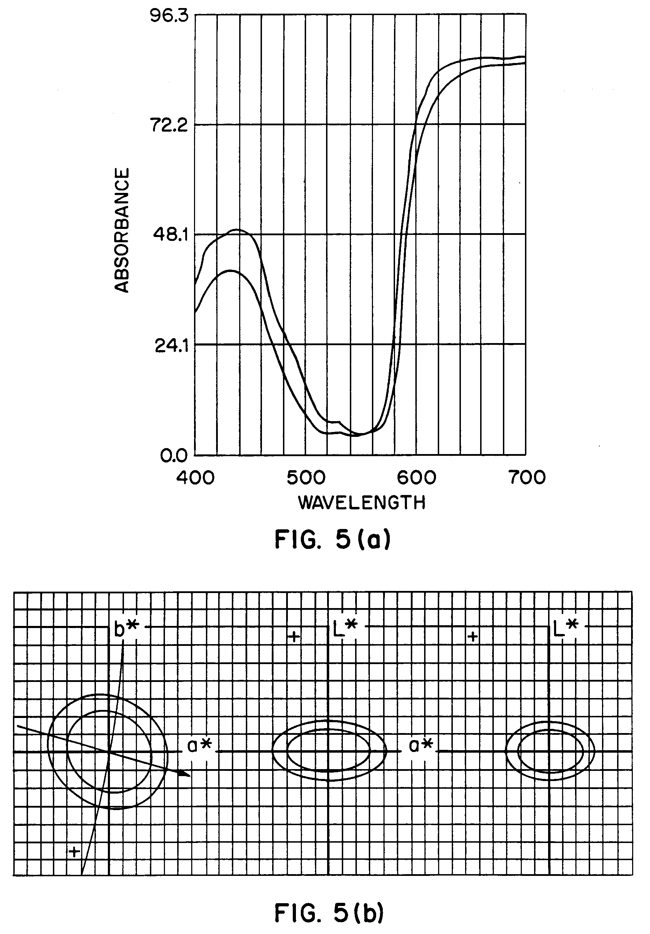

In the data presented in Tables I, II and III and Figures 3, 4 and 5, the following designations are used for pigment/substrate combinations. FD&C blue No. 1 laked on starch is designated B1 and FD&C blue No. 1 laked on aluminum hydrate is designated B2. FD&C green No. 5 laked on starch is designated G1 and FD&C green No. 5 laked on aluminum hydrate is designated G2. D&C red No. 28 laked on starch is designated R1 and D&C red No. 28 laked on aluminum hydrate is designated R2.

Dyes were laked on substrate at 10% load. Using a color spectrophotometer, measurements were taken of a color film made by dispersing 10% laked colors in an acrylic polymer solution.

Table IA, Table IIA and Table IIIA present the spectrophotomoter measurements for blue, green, and red dyes, respectively, laked onto either starch or aluminum hydrate. These results are graphically depicted in Figs. 3A, 4A and 5A respectively.

Tables IB, IIB and IIIB show the differences in color value for the various pigments laked onto starch or aluminum hydrate. For example, in Table IB the Delta E value 7.11 indicates that blue dye laked onto starch exhibited 7.11 units more color than blue dye laked onto aluminum hydrate. Similarly, the Delta E value in Table IIB indicates that green dye laked onto starch exhibited 5.15 units more color than green dye laked onto aluminum hydrate, and the Delta E value in Table IIIB indicated that red dye laked onto starch exhibited 6.15 units more color than red dye laked onto aluminum hydrate. The values presented in Tables IB, IIB and IIIB are graphically depicted in Figs. 3B, 4B and 5B respectively.

In summary, these experiments demonstrated the following. Blue dye laked onto aluminum hydrate was darker, less green, and less blue than blue dye laked onto starch. Green dye laked onto aluminum hydrate was lighter, less green and less blue than green dye laked onto starch. Red dye laked onto aluminum hydrate was lighter, less red and bluer than red dye laked onto starch. Accordingly, pigments laked onto starch appeared to be brighter and more intense than those same pigments laked onto aluminum hydrate.

**TABLE I**

| (a) | d/8° | B1 | B2 | (b) | | B1 | B2 |
|---|---|---|---|---|---|---|---|
| | 400 | 5.62 | 5.01 | | X | 9.13 | 7.84 |
| | 420 | 6.04 | 5.25 | | Y | 12.62 | 10.20 |
| | 440 | 24.03 | 18.88 | | Z | 33.11 | 27.92 |
| | 460 | 44.19 | 38.56 | | X | 0.1664 | 0.1706 |
| | 480 | 41.67 | 35.58 | | Y | 0.2300 | 0.2219 |
| | 500 | 31.89 | 25.17 | | | | |
| | 520 | 19.13 | 13.47 | | L* | 42.18 | 38.20 |
| | 540 | 8.66 | 6.31 | | a* | -21.63 | -15.77 |
| | 560 | 4.72 | 4.35 | | b* | -34.83 | -34.23 |
| | 580 | 4.11 | 3.95 | | | | |
| | 600 | 3.99 | 3.81 | | D10° | ΔL* | -3.98 |
| | 620 | 3.97 | 3.73 | | | Δa* | 5.86 |
| | 640 | 4.10 | 3.77 | | | Δb* | 0.60 |
| | 660 | 4.35 | 3.97 | | | ΔC* | -3.31 |
| | 680 | 4.98 | 4.32 | | | ΔE* | 7.11 |
| | 700 | 19.68 | 9.61 | | *REJECT* | | |

**TABLE II**

| (a) | d/8° | G1 | G2 | | | G1 | G2 |
|---|---|---|---|---|---|---|---|
| | 400 | 16.63 | 16.73 | | X | 19.19 | 19.87 |
| | 420 | 18.29 | 18.11 | | Y | 28.32 | 28.49 |
| | 440 | 24.70 | 23.19 | | Z | 35.38 | 32.34 |
| | 460 | 38.12 | 33.97 | | x | 0.2315 | 0.2462 |
| | 480 | 56.04 | 44.53 | | y | 0.3416 | 0.3530 |
| | 500 | 50.55 | 47.06 | | | | |
| | 520 | 41.58 | 40.70 | | L* | 60.18 | 60.33 |
| | 540 | 31.32 | 32.02 | | a* | -34.77 | -32.00 |
| | 560 | 22.94 | 24.62 | | b* | -6.83 | -2.49 |
| | 580 | 17.48 | 19.27 | | | | |
| | 600 | 13.12 | 15.26 | | D 10° | ΔL* | 0.15 |
| | 620 | 12.17 | 13.50 | | | Δa* | 2.77 |
| | 640 | 12.05 | 13.14 | | | Δb* | 4.34 |
| | 660 | 12.48 | 13.57 | | | ΔC* | -3.34 |
| | 680 | 19.15 | 16.97 | | | ΔE* | 5.15 |
| | 700 | 36.57 | 26.03 | | *REJECT* | | |

**TABLE III**

| (a) | d/8° | R1 | R2 | (b) | | R1 | R2 |
|---|---|---|---|---|---|---|---|
| | 400 | 30.59 | 36.37 | | X | 39.08 | 44.91 |
| | 420 | 37.91 | 46.95 | | Y | 22.05 | 26.61 |
| | 440 | 39.97 | 48.91 | | Z | 34.69 | 44.18 |
| | 460 | 32.81 | 42.16 | | x | 0.4078 | 0.3882 |
| | 480 | 17.07 | 26.04 | | y | 0.2302 | 0.2300 |
| | 500 | 8.03 | 13.41 | | | | |
| | 520 | 4.82 | 7.22 | | L* | 54.08 | 58.62 |
| | 540 | 4.34 | 5.43 | | a* | 70.02 | 68.15 |
| | 560 | 4.44 | 5.04 | | b* | -16.43 | -20.14 |
| | 580 | 16.99 | 26.62 | | | | |
| | 600 | 65.44 | 74.18 | | D 10° | ΔL* | 4.53 |
| | 620 | 78.88 | 83.90 | | | Δa* | -1.87 |
| | 640 | 82.78 | 86.30 | | | Δb* | -3.71 |
| | 660 | 84.73 | 87.08 | | | ΔC* | -0.86 |
| | 680 | 85.47 | 87.20 | | | ΔE* | 6.15 |
| | 700 | 86.13 | 87.50 | | *REJECT* | | |

## Claims

1. A composition comprising colorant-containing particles having a coating comprising propylene glycol and a carrier component selected from the group consisting of carriers suitable for food, drug and cosmetic preparations and mixtures thereof, wherein the colorant-containing particles comprise an organic dye laked on a suitable substrate.

2. A composition according to claim 1 wherein the dye is suitable for use in food, drug and cosmetic preparations.

3. A composition according to claim 1 wherein the colorant in the colorant-containing particles is susceptible to ultraviolet degradation.

4. A composition according to claim 1 wherein the carrier component of the coating is titanium dioxide.

5. A composition according to claim 4 wherein the average size of the carrier component of the coating ranges from .1 to .3 micrometer.

6. A composition according to claim 1 wherein the average particle size of the coated colorant-containing particle is 0.05 to .4 micrometer.

7. A process for preparing a composition according to claim 1, said process comprising the steps of: mixing said colorant-containing particles with particles of a carrier selected from the group consisting of carriers suitable for food, drug and cosmetic preparations and mixtures thereof; and contacting the resulting mixture of particles with propylene glycol whereby colorant-containing particles coated with the carrier and propylene glycol are formed.

8. A process according to claim 7, wherein the colorant is suitable for use in food or drug or cosmetic preparations.

9. A process according to claim 7 wherein the colorant-containing particles have an average particle size ranging from .05 to .4 micrometer prior to coating.

10. A process according to claim 7 wherein the colorant-containing particles are mixed with micronized particles of the carrier to form a homogenous blend.

11. A process according to claim 7 wherein the carrier is micronized titanium dioxide having a particle size ranging from 0.1 to 0.3 micrometer.

12. A process according to claim 7 wherein the propylene glycol is sprayed onto the mixture of colorant-containing particles and micronized carrier.

13. Use of a coating comprising propylene glycol and a carrier selected from the group consisting of carriers suitable for food, drug and cosmetic preparations and mixtures thereof for improving the stability of colorant-containing particles which are degraded by ultraviolet light.

14. Use of propylene glycol and a carrier selected from the group consisting of carriers suitable for food, drug and cosmetic preparations and mixtures thereof for improving the sun protection factor (SPF) of a cosmetic composition comprising a colorant-containing particle.

15. Use according to claim 14 wherein the carrier is titanium dioxide.

16. Use of a coating comprising propylene glycol and uncolored particles of a substrate compound for improving the photostability of a dye suitable for food, cosmetic or drug preparations, laked onto a particle consisting of the substrate compound suitable for food, cosmetic or drug preparations in a dye-laked particle.

17. Use according to claim 16 in which the substrate compound is titanium dioxide.

18. Use according to claim 16 in which the substrate compound is zinc oxide.

19. Use according to claim 16 in which the substrate compound is mica.

20. Use according to claim 16 in which the substrate compound is starch.

21. The composition of claim 1 in which the substrate is selected from the group consisting of starch and mica.

22. The process of claim 7 in which the substrate is selected from the group consisting of starch and mica.

23. Use according to claim 13 in which the particles comprise a substrate selected from the group consisting of starch and mica.

24. Use according to claim 14 in which the particles comprise a substrate selected from the group consisting of starch and mica.

25. Use according to claim 16 in which the substrate is selected from the group consisting of starch and mica.

## Patentansprüche

1. Zusammensetzung, umfassend Farbmittel-hältige Teilchen mit einer Beschichtung, welche Propylenglycol und eine Trägerkomponente ausgewählt aus Trägern, die für Lebensmittel-, Arzneimittel- und Kosmetika-Zubereitungen geeignet sind, und Mischungen davon umfaßt, wobei die Farbmittel-hältigen Teilchen einen auf ein geeignetes Substrat aufgebrachten bzw. lackierten organischen Farbstoff umfassen.

2. Zusammensetzung nach Anspruch 1, wobei der Farbstoff für die Verwendung in Lebensmittel-, Arzneimittel- und Kosmetika-Zubereitungen geeignet ist.

3. Zusammensetzung nach Anspruch 1, wobei das Farbmittel in den Farbmittel-hältigen Teilchen für einen Abbau durch Ultraviolettlicht anfällig ist.

4. Zusammensetzung nach Anspruch 1, wobei die Trägerkomponente der Beschichtung Titandioxid ist.

5. Zusammensetzung nach Anspruch 4, wobei die durchschnittliche Größe der Trägerkomponente der Beschichtung von 0,1 bis 0,3 Mikrometer reicht.

6. Zusammensetzung nach Anspruch 1, wobei die durchschnittliche Teilchengröße des beschichteten Farbmittel-hältigen Teilchens 0,05 bis 0,4 Mikrometer beträgt.

7. Verfahren zur Herstellung einer Zusammensetzung nach Anspruch 1, welches Verfahren die folgenden Schritte umfaßt: Mischen der Farbmittel-hältigen Teilchen mit Teilchen eines Trägers ausgewählt aus Trägern, die für Lebensmittel-, Arzneimittel- und Kosmetika-Zubereitungen geeignet sind, und Mischungen davon; und Kontaktieren der resultierenden Teilchenmischung mit Propylenglycol, wodurch Farbmittel-hältige Teilchen, beschichtet mit dem Träger und Propylenglycol, gebildet werden.

8. Verfahren nach Anspruch 7, wobei das Farbmittel für die Verwendung in Lebensmittel- oder Arzneimittel- oder Kosmetika-Zubereitungen geeignet ist.

9. Verfahren nach Anspruch 7, wobei die Farbmittel-hältigen Teilchen vor dem Beschichten eine durchschnittliche Teilchengröße von 0,05 bis 0,4 Mikrometer aufweisen.

10. Verfahren nach Anspruch 7, wobei die Farbmittel-hältigen Teilchen mit mikronisierten Teilchen des Trägers vermischt werden, um eine homogene Mischung zu bilden.

11. Verfahren nach Anspruch 7, wobei der Träger mikronisiertes Titandioxid mit einer Teilchengröße von 0,1 bis 0,3 Mikrometer ist.

12. Verfahren nach Anspruch 7, wobei das Propylenglycol auf die Mischung von Farbmittelhältigen Teilchen und mikronisiertem Träger gesprüht wird.

13. Verwendung einer Beschichtung, umfassend Propylenglycol und einen Träger, ausgewählt aus Trägern, die für Lebensmittel-, Arzneimittel- und Kosmetika-Zubereitungen geeignet sind, und Mischungen davon zur Verbesserung der Beständigkeit von Farbmittel-hältigen Teilchen, welche durch ultraviolettes Licht abgebaut werden.

14. Verwendung von Propylenglycol und einem Träger ausgewählt aus Trägern, die für Lebensmittel-, Arzneimittel- und Kosmetika-Zubereitungen geeignet sind, und Mischungen davon zur Verbesserung des Sonnenschutzfaktors (SPF) einer Kosmetikum-Zusammensetzung, welche ein Farbmittel-hältiges Teilchen umfaßt.

15. Verwendung nach Anspruch 14, wobei der Träger Titandioxid ist.

16. Verwendung einer Beschichtung umfassend Propylenglycol und ungefärbte Teilchen einer Substratverbindung zur Verbesserung der Lichtbeständigkeit eines Farbstoffes, welcher für Lebensmittel-, Kosmetika- oder Arzneimittel-Zubereitungen geeignet und auf ein Teilchen aufgebracht bzw. lackiert ist, das aus der für Lebensmittel-, Kosmetika- oder Arzneimittelzubereitungen geeigneten Substratverbindung in einem mit Farbstoff lackiertem Teilchen besteht.

17. Verwendung nach Anspruch 16, wobei die Substratverbindung Titandioxid ist.

18. Verwendung nach Anspruch 16, wobei die Substratverbindung Zinkoxid ist.

19. Verwendung nach Anspruch 16, wobei die Substratverbindung Glimmer ist.

20. Verwendung nach Anspruch 16, wobei die Substratverbindung Stärke ist.

21. Zusammensetzung von Anspruch 1, wobei das Substrat aus Stärke und Glimmer ausgewählt ist.

22. Verfahren von Anspruch 7, wobei das Substrat aus Stärke und Glimmer ausgewählt ist.

23. Verwendung nach Anspruch 13, wobei die Teilchen ein Substrat umfassen, das aus Stärke und Glimmer ausgewählt ist.

24. Verwendung nach Anspruch 14, wobei die Teilchen ein Substrat umfassen, das aus Stärke und Glimmer ausgewählt ist.

25. Verwendung nach Anspruch 16, wobei das Substrat aus Stärke und Glimmer ausgewählt ist.

## Revendications

1. Composition comportant des particules contenant un colorant, recouvertes d'un revêtement comportant du propylèneglycol et un support choisi dans le groupe constitué des supports convenant pour des préparations alimentaires, médicamenteuses et cosmétiques et des mélanges de ces supports, dans laquelle les particules contenant un colorant comportent un colorant organique laqué sur un substrat approprié.

2. Composition selon la revendication 1, dans laquelle le colorant convient pour une utilisation dans des préparations alimentaires, médicamenteuses et cosmétiques.

3. Composition selon la revendication 1, dans laquelle le colorant des particules contenant un colorant est susceptible de se dégrader sous l'action des ultraviolets.

4. Composition selon la revendication 1, dans laquelle le support du revêtement est le dioxyde de titane.

5. Composition selon la revendication 4, dans laquelle la taille moyenne du support du revêtement est comprise entre 0,1 et 0,3 micromètres.

6. Composition selon la revendication 1, dans laquelle la taille moyenne des particules contenant un colorant revêtues est comprise entre 0,05 à 0,4 micromètres.

7. Procédé pour la préparation d'une composition selon la revendication 1, ledit procédé comportant les étapes consistant à:
mélanger lesdites particules contenant un colorant avec des particules d'un support choisi dans le groupe constitué des supports convenant pour des préparations alimentaires, médicamenteuses et cosmétiques et des mélanges de ces supports,
et mettre le mélange de particules ainsi obtenu en contact avec du propylèneglycol pour former des particules contenant un colorant revêtues par le substrat et le propylèneglycol.

8. Procédé selon la revendication 7, dans lequel le colorant convient pour une utilisation dans des préparations alimentaires, médicamenteuses ou cosmétiques.

9. Procédé selon la revendication 7, dans lequel, avant le revêtement, la taille moyenne des particules contenant un colorant est comprise entre 0,05 et 0,4 micromètres.

10. Procédé selon la revendication 7, dans lequel les particules contenant un colorant sont mélangées à des particules micronisées du support, de manière à former un mélange homogène.

11. Procédé selon la revendication 7, dans lequel le support est constitué de particules micronisées de dioxyde de titane dont la taille moyenne est comprise entre 0,1 et 0,3 micromètres.

12. Procédé selon la revendication 7, dans lequel le propylèneglycol est pulvérisé sur le mélange de particules contenant un colorant et de supports micronisés.

13. Utilisation d'un revêtement comportant du propylèneglycol et un support choisi dans le groupe constitué des supports convenant pour des préparations alimentaires, médicamenteuses et cosmétiques et des mélanges de ces supports, en vue d'améliorer la stabilité des particules contenant un colorant qui sont dégradées par la lumière ultraviolette.

14. Utilisation de propylèneglycol et d'un support choisi dans le groupe constitué des supports convenant pour des préparations alimentaires, médicamenteuses et cosmétiques et des mélanges de ceux-ci, en vue d'améliorer l'indice de protection solaire ("sun protection factor" - SPF) d'une composition cosmétique comportant des particules contenant un colorant.

15. Utilisation selon la revendication 14, dans laquelle le support est le dioxyde de titane.

16. Utilisation d'un revêtement comportant du propylèneglycol et des particules non colorées d'un substrat, en vue d'améliorer la photostabilité d'un colorant convenant pour des préparations alimentaires, cosmétiques ou médicamenteuses, laqué sur des particules constituées du substrat convenant pour des préparations alimentaires, cosmétiques ou médicamenteuses pour former des particules laquées par un colorant.

17. Utilisation selon la revendication 16, dans laquelle le substrat est le dioxyde de titane.

18. Utilisation selon la revendication 16, dans laquelle le substrat est l'oxyde de zinc.

19. Utilisation selon la revendication 16, dans laquelle le substrat est le mica.

20. Utilisation selon la revendication 16, dans laquelle le substrat est l'amidon.

21. Composition selon la revendication 1, dans laquelle le substrat est choisi dans le groupe constitué de l'amidon et du mica.

22. Procédé selon la revendication 7, dans laquelle le substrat est choisi dans le groupe constitué de l'amidon et du mica.

23. Utilisation selon la revendication 13, dans laquelle les particules comportent un substrat choisi dans le groupe constitué de l'amidon et du mica.

24. Utilisation selon la revendication 14, dans laquelle les particules comportent un substrat choisi dans le groupe constitué de l'amidon et du mica.

25. Utilisation selon la revendication 16, dans laquelle le substrat est choisi dans le groupe constitué de l'amidon et du mica.
